# EUROPEAN PATENT APPLICATION

(11) **EP 4 822 735 A1**
(43) Date of publication of application: **14.10.2026**
(21) Application number: 24901007.5
(22) Date of filing: 03.12.2024
(51) Int. Cl.: H01J 35/16, H01J 35/08, H01J 35/18, A61B 6/40, A61B 6/03

(54) **X-RAY TUBE AND SYSTEM INCLUDING SAME**

(30) Priority: 06.12.2023 KR 20230175154
(71) Applicant: Koh Young Technology Inc, Seoul 08588 (KR)
(72) Inventor: KANG, Jung Su, Suwon-si Gyeonggi-do 16713 (KR); KIM, Young Kwang, Anyang-si Gyeonggi-do 13964 (KR); JO, Eun Ha, Gwangmyeong-si Gyeonggi-do 14313 (KR); JEONG, Pil Soo, Yongin-si Gyeonggi-do 16812 (KR)
(74) Representative: Meissner Bolte Partnerschaft mbB
(86) International application number: PCT/KR2024/019517
(87) International publication number: WO 2025/121821

(57) **Abstract**

According to an embodiment of the present disclosure, an X-ray tube includes: an electron gun; an anode including a distal end portion onto which electrons emitted from the electron gun are irradiated and from which X-rays are emitted, the anode being surrounded by a conductive material; a case including a first receptacle formed of the conductive material and connected to the electron gun, and a second receptacle coupled to the first receptacle and defining, together with the first receptacle, an internal space for accommodating the anode, the first receptacle including a side wall connected to the electron gun and a lower wall formed perpendicular to the side wall; and an irradiation window formed in the lower wall of the first receptacle and configured to extract the X-rays emitted from the distal end portion to the outside of the case.

## Description

### TECHNICAL FIELD

The present disclosure relates to an X-ray tube and a system including the same.

The present disclosure was derived from research conducted as part of the "Mid-sized Enterprise DNA Convergence Industry-Academia Cooperation Project" supported by the Ministry of Trade, Industry and Resources (MOTIR) and the Korea Institute for Advancement of Technology (KIAT).

### BACKGROUND

X-rays are electromagnetic waves having excellent penetrability with respect to objects, and may be used for non-destructive and non-contact observation of the internal structure of an object or a human body. Electrons emitted from a cathode are accelerated by an anode and collide with an anode tungsten target, and only a portion (less than about 1%) of electrical energy applied to an X-ray tube is emitted as X-rays from the anode tungsten target. The X-rays emitted from the anode are extracted to the outside of the X-ray tube and may be used for non-destructive and non-contact observation of the internal structure of an object or a human body.

### DISCLOSURE

### TECHNICAL PROBLEM

Various embodiments of the present disclosure provide a technology related to an X-ray tube and a system including the same.

### TECHNICAL SOLUTION

One aspect of the present disclosure provides embodiments of an X-ray tube. According to an embodiment of the present disclosure, an X-ray tube includes: an electron gun; an anode including a distal end portion onto which electrons emitted from the electron gun are irradiated and from which X-rays are emitted, the anode being surrounded by a conductive material; a case including a first receptacle formed of the conductive material and connected to the electron gun, and a second receptacle coupled to the first receptacle and defining, together with the first receptacle, an internal space for accommodating the anode, the first receptacle including a side wall connected to the electron gun and a lower wall formed perpendicular to the side wall; and an irradiation window formed in the lower wall of the first receptacle and configured to extract the X-rays emitted from the distal end portion to the outside of the case, wherein a movement direction of the X-rays emitted from the distal end portion crosses a movement direction of the electrons emitted from the electron gun.

In an embodiment, at a position corresponding to a receptacle coupling portion at which the first receptacle and the second receptacle are coupled to each other, a distance from an outer peripheral surface of the anode to the first receptacle is greater than a distance from the outer peripheral surface of the anode to the second receptacle.

In an embodiment, the anode includes: a first portion located on a side of the second receptacle in the case; a second portion located on a side of the irradiation window of the first receptacle in the case; and a third portion located between the first portion and the second portion, wherein the first portion, the second portion, and the third portion are arranged side by side in a longitudinal direction, and a circumference of an outer peripheral surface of the third portion is smaller than a circumference of an outer peripheral surface of the first portion and a circumference of an outer peripheral surface of the second portion.

In an embodiment, the circumference of the outer peripheral surface of the first portion and the circumference of the outer peripheral surface of the second portion are identical.

In an embodiment, the second receptacle is formed of an insulator.

In an embodiment, the second receptacle is formed of ceramic.

In an embodiment, the irradiation window is integrally formed with the first receptacle.

In an embodiment, the irradiation window is formed of at least one selected from Be, Al, and Ti.

In an embodiment, the distal end portion is disposed to be inclined with respect to an emission direction of the electrons emitted from the electron gun.

In another aspect of the present disclosure, the distal end portion is disposed to be inclined with respect to an emission direction of the electrons emitted from the electron gun.

One aspect of the present disclosure provides embodiments of an X-ray system. According to an embodiment of the present disclosure, an X-ray tube system includes: a plurality of X-ray tubes, each including: an electron gun; an anode including a distal end portion onto which electrons emitted from the electron gun are irradiated and from which X-rays are emitted, the anode being surrounded by a conductive material; a case including a first receptacle formed of the conductive material and connected to the electron gun, and a second receptacle coupled to the first receptacle and defining, together with the first receptacle, an internal space for accommodating the anode, the first receptacle including a side wall connected to the electron gun and a lower wall formed perpendicular to the side wall; and an irradiation window formed in the lower wall of the first receptacle and configured to extract the X-rays emitted from the distal end portion to the outside of the case; a slit through which each of the X-rays passes and which adjusts an irradiation field of each of the X-rays; and a detector configured to receive each of the X-rays emitted from the plurality of X-ray tubes, wherein the plurality of X-ray tubes are arranged side by side on a plane parallel to the detector, and a movement direction of each of the X-rays crosses a movement direction of each of the electrons emitted from the electron gun.

In an embodiment, the plurality of X-ray tubes include three or more X-ray tubes configured to irradiate the detector with the respective X-rays in parallel with one another.

In an embodiment, intensities of the respective X-rays are different from one another.

In an embodiment, among the three or more X-ray tubes, an intensity of X-rays emitted from an X-ray tube located between two other X-ray tubes is greatest.

### ADVANTAGEOUS EFFECTS

According to one embodiment of the present disclosure, a plurality of X-ray tubes can be arranged more densely inside an X-ray monoblock.

According to one embodiment of the present disclosure, electric field directions of the X-ray tubes can be arranged identically.

According to one embodiment of the present disclosure, an X-ray exposure amount for each portion of an object or a human body can be easily adjusted.

According to one embodiment of the present disclosure, a more accurate diagnostic image can be provided.

Effects according to the technology of the present disclosure are not limited to the above-mentioned effects, and other effects not mentioned herein will be clearly understood by those skilled in the art from the description of the present disclosure.

### DESCRIPTION OF DRAWINGS

FIG. 1 is a perspective view of an X-ray tube according to an embodiment of the present disclosure.
FIG. 2 is a cross-sectional view of the X-ray tube taken along line A-A' in FIG. 1.
FIG. 3 is a schematic view of an X-ray tube system including a plurality of X-ray tubes according to an embodiment of the present disclosure.
FIG. 4 is a cross-sectional view of an X-ray tube system including a plurality of X-ray tubes according to an embodiment of the present disclosure.
FIG. 5 is a top perspective view of an X-ray tube according to an embodiment of the present disclosure.
FIG. 6 is a rear perspective view of an X-ray tube according to an embodiment of the present disclosure.
FIG. 7 is a cross-sectional perspective view of the X-ray tube taken along line B-B' in FIG. 5.
FIG. 8 is a schematic view illustrating a focal track of a light source position of an X-ray tube according to an embodiment of the present disclosure.

### MODE FOR INVENTION

Various embodiments described herein are exemplified for the purpose of clearly explaining the technical idea of the present disclosure, and are not intended to limit the present disclosure to specific embodiments.

All terms used herein, including technical or scientific terms, have meanings that are generally understood by those skilled in the art to which the present disclosure pertains, unless otherwise specified. All terms used herein are selected for the purpose of further clarifying the present disclosure and are not selected to limit the scope of rights under the present disclosure.

Expressions used herein, such as "include," "comprise," "have," etc., should be understood as open-ended terms connoting the possibility of inclusion of other embodiments, unless otherwise stated in the phrase or sentence containing such expressions.

A singular expression used herein may include meanings of plurality, unless otherwise mentioned in the context, and this also applies to a singular expression recited in the claims.

The expressions "a first," "a second," used herein are used to distinguish one object from another object in referring to a plurality of homogeneous objects, unless the context dictates otherwise, and do not limit the order or the importance of the objects.

Herein, where it is stated that a component is "connected" or "coupled" to another component, it should be understood that said component may be directly connected or coupled to the other component, or that component may be connected or coupled through a new alternative component.

The dimensions and numbers described in the present disclosure are not limited to the dimensions and numbers described. Unless otherwise specified, such dimensions and numbers may be understood to mean the values described and equivalent ranges including them. For example, the dimension "50 mm" described in the present disclosure may be understood to include "about 50 mm."

Hereinafter, various embodiments of the present disclosure will be described with reference to the accompanying drawings. In the accompanying drawings and the description of the drawings, identical or substantially equivalent elements may be assigned with identical reference numerals. Further, in the following description of various embodiments, redundant descriptions of the identical or relevant elements may be omitted. However, this does not imply that such elements are not included in the embodiments.

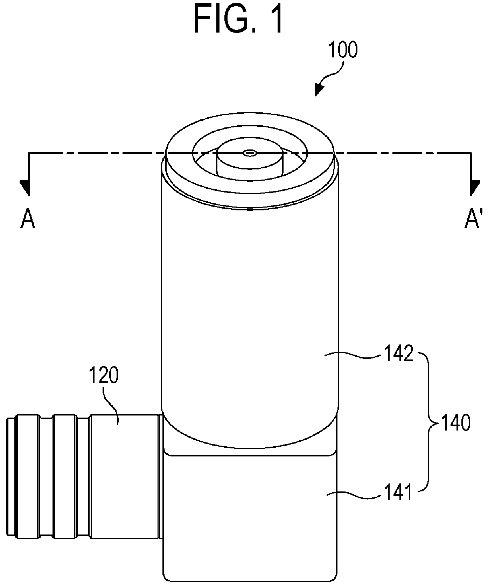

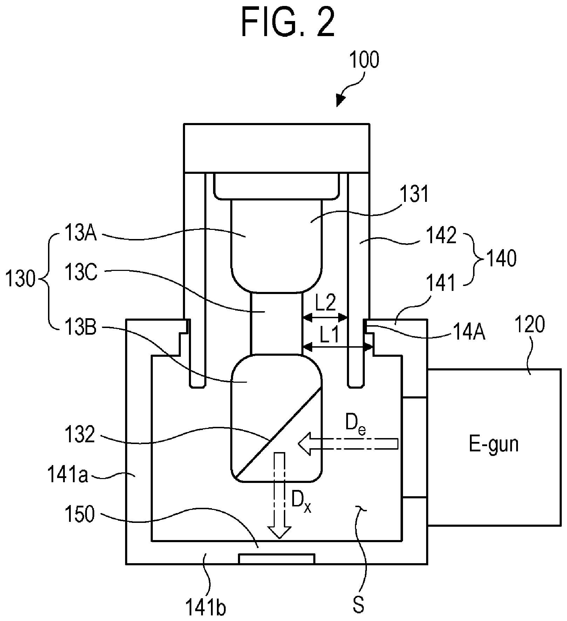

FIG. 1 is a perspective view of an X-ray tube 100 according to an embodiment of the present disclosure. FIG. 2 is a cross-sectional view of the X-ray tube 100 taken along line A-A' in FIG. 1. Referring to FIGS. 1 and 2, the X-ray tube 100 may include an electron gun 120, an anode 130, a case 140, and an irradiation window 150.

In one embodiment, the electron gun 120 generates electrons and emits the electrons toward the anode 130, and a cold cathode electron tube configured to emit electrons by an electric field difference formed due to a potential difference may be installed therein. Internal spaces of the electron gun 120 and the case 140 communicate with each other and may be sealed from the outside of the X-ray tube 100.

In one embodiment, the anode 130 may be formed as a positive electrode. The anode 130 may be surrounded by a conductive material. The anode 130 may have a shape extending in a direction perpendicular to an emission direction De of electrons emitted from the electron gun 120 (hereinafter, referred to as a longitudinal direction). Specifically, the anode 130 may include a first portion 13A located on a side of a second receptacle 142 of the case 140, a second portion 13B located on an opposite side of the first portion 13A, that is, on a side of a lower wall 141b of a first receptacle 141, and a third portion 13C located between the first portion 13A and the second portion 13B. The first portion 13A, the third portion 13C, and the second portion 13B may be sequentially arranged side by side along the longitudinal direction. Circumferences of outer peripheral surfaces of the first portion 13A, the second portion 13B, and the third portion 13C may be different from one another. Specifically, the circumference of the outer peripheral surface of the third portion 13C may be smaller than the circumferences of the outer peripheral surfaces of the first portion 13A and the second portion 13B. When a gap between the anode 130 and the case 140 is narrow, a pinhole defect through which electrons or high-energy materials penetrate a wall of the case 140 may occur. Accordingly, by forming the circumference of the outer peripheral surface of the third portion 13C, which is disposed closest to the second receptacle 142 of the case 140, to be the smallest, the gap between the anode 130 and the case 140 may be secured, thereby preventing the pinhole defect. In this case, the circumferences of the outer peripheral surfaces of the first portion 13A and the second portion 13B may be identical. That is, only a middle portion (e.g., the third portion 13C) of the cylindrical anode 130 may be formed to be concave.

In one embodiment, the anode 130 may include a base end portion 131 and a distal end portion 132. The base end portion 131 may correspond to a longitudinal column on which the first portion 13A, the second portion 13B, and the third portion 13C are disposed. A high voltage may be applied to the base end portion 131. The distal end portion 132 may correspond to an end portion formed on the side of the second portion 13B of the base end portion 131 in the anode 130. The electron gun 120 may emit electrons toward the distal end portion 132. As the incident electrons collide with the distal end portion 132 at high speed, kinetic energy of the electrons may be converted into X-rays and thermal energy. Due to such energy conversion, X-rays may be emitted from the distal end portion 132. The X-rays generated from the distal end portion 132 may pass through the irradiation window 150 and be irradiated outward. Referring to FIG. 2, the electron emission direction De and a movement direction Dx of the X-rays may be perpendicular to each other. In order for the electron emission direction De and the X-ray movement direction Dx to be perpendicular to each other, the distal end portion 132 may be disposed to be inclined with respect to the electron emission direction De. That is, a reflective surface of the distal end portion 132 may be disposed to be inclined with respect to the electron emission direction De.

In one embodiment, the case 140 may include the first receptacle 141 and the second receptacle 142. The first receptacle 141 may be formed of a conductive material and may surround the periphery of the anode 130, particularly the periphery of the second portion 13B including the distal end portion 132. The first receptacle 141 may include a side wall 141a surrounding an outer peripheral surface of the anode 130 and the lower wall 141b formed perpendicular to a direction in which X-rays are extracted outward from the anode 130. The lower wall 141b may be formed perpendicular to the side wall 141a. The first receptacle 141 may be connected to the electron gun 120.

In one embodiment, the first receptacle 141 and the second receptacle 142 may be coupled to each other to define an internal space S for accommodating the anode 130. The case 140 may further include a receptacle coupling portion 14A at which the first receptacle 141 and the second receptacle 142 contact each other. At a position corresponding to the receptacle coupling portion 14A, a distance L1 from the outer peripheral surface of the anode 130 to the first receptacle 141 may be greater than a distance L2 from the outer peripheral surface of the anode 130 to the second receptacle 142. Specifically, at the position corresponding to the receptacle coupling portion 14A, the first receptacle 141 may be formed to surround the second receptacle 142. Since the first receptacle 141 is formed to surround the second receptacle 142, even when energy in the internal space S increases, an electric field may be easily maintained. In addition, since the first receptacle 141 is formed to surround the second receptacle 142, the internal space S in a region surrounding the distal end portion 132 of the anode 130 may be widened. Accordingly, the anode 130 having a larger diameter (circumference) may be disposed inside the case 140.

In one embodiment, the first receptacle 141 may be made of metal. Specifically, the first receptacle 141 may be formed of KOVAR, which is an alloy of iron, nickel, and cobalt. As the first receptacle 141 formed of metal is grounded, secondary electrons and/or scattered electrons generated by electron beam generation may be discharged through the corresponding ground portion, thereby enabling stable operation of an X-ray source. Accordingly, the X-ray tube 100 according to one embodiment may stably extract high-voltage and high-output X-rays.

In one embodiment, the second receptacle 142 may be formed of an insulator. Specifically, the second receptacle 142 may be formed of ceramic. Since the second receptacle 142 is formed of an insulator, specifically ceramic, an insulation effect may be maximized and a vacuum state of the internal space S may be easily maintained.

In one embodiment, the irradiation window 150 may serve to extract X-rays emitted from the distal end portion 132 of the anode 130 to the outside of the case 140, that is, to the outside of the X-ray tube 100. The irradiation window 150 may be formed in the lower wall 141b of the first receptacle 141. In one embodiment, the irradiation window 150 may be formed of the same material as the first receptacle 141. In one embodiment, the irradiation window 150 may be integrally formed with the first receptacle 141. When the irradiation window 150 is integrally formed of the same material as the first receptacle 141, manufacturing cost and time of the X-ray tube may be reduced. Depending on the embodiments, the irradiation window 150 may be formed (manufactured) of at least one selected from metals such as Be, Al, or Ti. Since an absorption rate of X-rays by the irradiation window 150 varies depending on the density of the material forming the irradiation window 150, a practitioner may appropriately select the material of the irradiation window 150 as necessary.

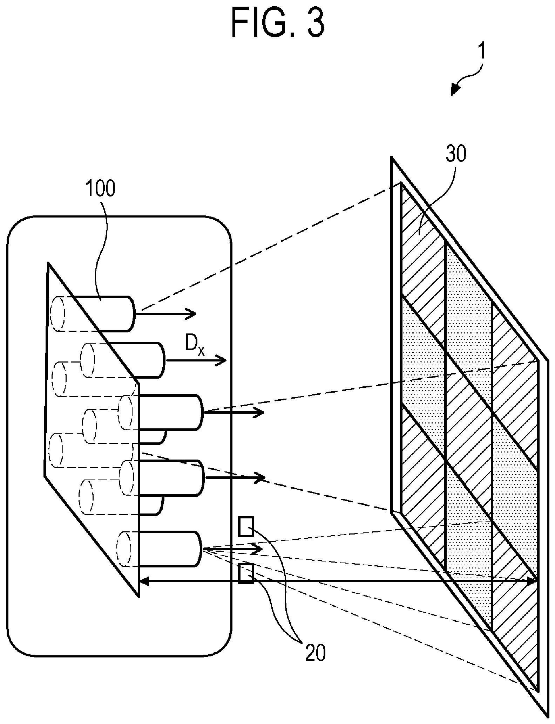

FIG. 3 is a schematic view of an X-ray tube system 1 including a plurality of X-ray tubes 100 according to an embodiment of the present disclosure. Referring to FIG. 3, the X-ray tube system 1 may include a plurality of X-ray tubes 100 according to an embodiment of the present disclosure. In addition, the X-ray tube system 1 may include a slit 20 and a detector 30.

In one embodiment, the plurality of X-ray tubes 100 may be arranged side by side on a plane parallel to the detector 30. More specifically, the plurality of X-ray tubes 100 may be arranged such that lower surfaces (e.g., the lower wall 141b of FIG. 2) of first receptacles (e.g., the first receptacle 141 of FIG. 2) are disposed on a plane parallel to the detector 30. Intensities of X-rays extracted from the respective X-ray tubes 100 may be different from one another.

In one embodiment, the slit 20 may variably adjust (limit) an irradiation field of X-rays extracted from the X-ray tube 100 to limit an area irradiated with X-rays on the detector 30. The irradiation field may be variably determined according to an imaging distance (a distance from the X-ray tube 100 to the detector 30). In addition, the X-rays may be irradiated such that X-ray images irradiated by the respective X-ray tubes 100 are connected to one another at edges thereof.

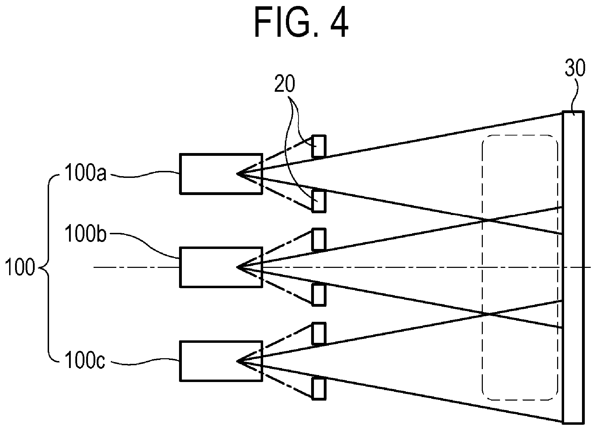

FIG. 4 is a cross-sectional view of the X-ray tube system 1 including the plurality of X-ray tubes 100 according to an embodiment of the present disclosure. Referring to FIG. 4, the plurality of X-ray tubes 100 may irradiate X-rays toward the detector 30. The X-rays irradiated toward the detector 30 may pass through the slit 20, and an irradiation field thereof may be adjusted (limited) by the slit 20 and irradiated onto the detector 30. The plurality of X-ray tubes 100 may include at least three X-ray tubes 100. For example, the X-ray tubes 100 may include a first X-ray tube 100a, a second X-ray tube 100b, and a third X-ray tube 100c.

In one embodiment, intensities of X-rays irradiated from the first X-ray tube 100a, the second X-ray tube 100b, and the third X-ray tube 100c may be different from one another. Specifically, the intensity of X-rays emitted from the X-ray tube 100 located between two X-ray tubes 100 may be greatest. For example, among the first X-ray tube 100a, the second X-ray tube 100b, and the third X-ray tube 100c, the intensity of X-rays emitted from the second X-ray tube 100b located between the first X-ray tube 100a and the second X-ray tube 100c may be greatest. Accordingly, during X-ray diagnostic imaging, efficient X-ray imaging may be enabled by adjusting intensities of extracted X-rays according to a density of an imaging target disposed between the plurality of X-ray tubes 100 and the detector 30. That is, low-dose X-ray diagnostic imaging may be enabled. In addition, since the plurality of X-ray tubes 100 according to one embodiment are arranged in parallel, even when irradiation angles of X-rays extracted from the respective X-ray tubes 100 are limited, X-rays may be irradiated onto a relatively wide area of the detector 30. Accordingly, an imaging distance (a distance from the X-ray tubes 100 to the detector 30) may be reduced, thereby enabling efficient utilization of space, and high-quality images or videos may be acquired even with low X-ray output.

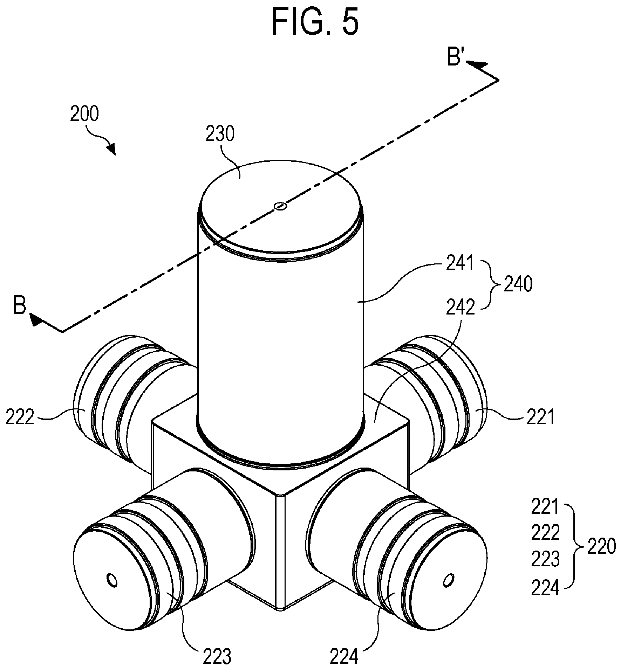

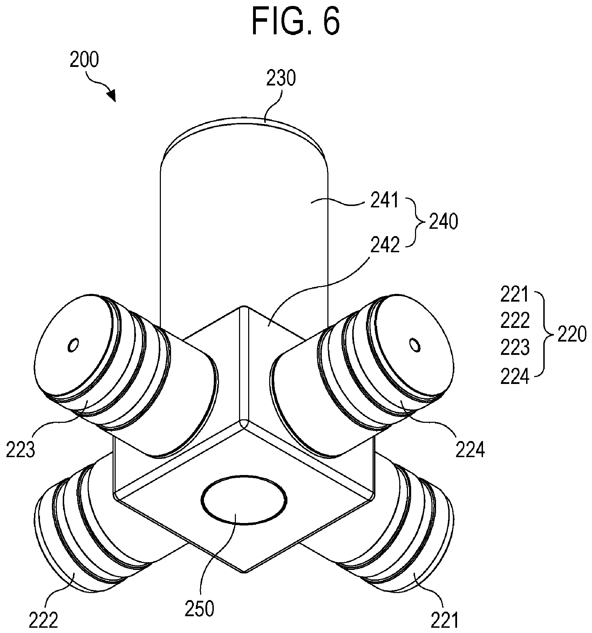

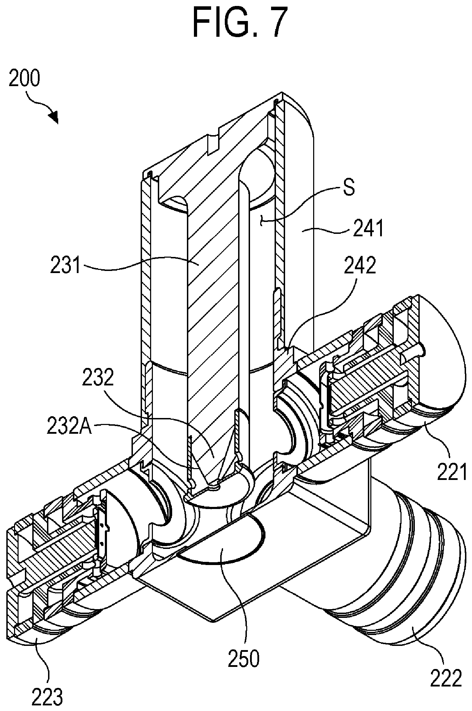

FIG. 5 is a top perspective view of an X-ray tube 200 according to an embodiment of the present disclosure. FIG. 6 is a rear perspective view of the X-ray tube 200 according to an embodiment of the present disclosure. FIG. 7 is a cross-sectional perspective view of the X-ray tube 200 taken along line B-B' in FIG. 5. Referring to FIGS. 5 to 7, the X-ray tube 200 may include a plurality of electron guns 220, an anode 230, a case 240, and an irradiation window 250.

In one embodiment, the electron guns 220 generate electrons and emit the electrons toward the anode 230, and a cold cathode electron tube configured to emit electrons by an electric field difference formed due to a potential difference may be installed therein. The internal spaces of the electron guns 220 and the case 240 communicate with each other and may be sealed from the outside of the X-ray tube 200. The plurality of electron guns 220 may be radially arranged around the anode 130. Angles between the plurality of electron guns may be constant. Specifically, when the number of the plurality of electron guns 220 is n, an angle between the respective electron guns 220 may be 360/n degrees (°). For example, when the number of electron guns 220 is 2, the angle between the electron guns 220 may be 180 degrees. For example, when the number of electron guns 220 is 3, the angle between the respective electron guns 220 may be 120 degrees. For example, when the number of electron guns 220 is 4, the angle between the respective electron guns 220 may be 90 degrees. In particular, when X-ray imaging is performed using the X-ray tube 200 including four or more electron guns 220, precise and stereoscopic CT images or photographs may be obtained without mechanical shaking through X-ray images acquired from four or more views. For example, referring to FIGS. 5 to 7, the electron guns 220 may include a first electron gun 221, a second electron gun 222, a third electron gun 223, and a fourth electron gun 224. An angle between each of the first electron gun 221, the second electron gun 222, the third electron gun 223, and the fourth electron gun 224 is 90 degrees.

In one embodiment, the anode 230 may be formed as a positive electrode. The anode 230 may be surrounded by a conductive material. The anode 230 may have a shape extending in a direction perpendicular to a movement direction of electrons emitted from the electron gun 220 (e.g., the movement direction De of FIG. 2) (hereinafter, referred to as a longitudinal direction). The anode 230 may include a base end portion 231 and a distal end portion 232. The base end portion 231 may correspond to a longitudinal columnar portion, and a high voltage may be applied to the base end portion 231. The distal end portion 232 may correspond to a terminal end of the base end portion 231 in the anode 230. Electrons may be emitted from the electron guns 220 toward the distal end portion 232, and energy conversion may occur due to electrons incident on the distal end portion 232, thereby generating X-rays from the distal end portion 232. The X-rays generated from the distal end portion 232 may pass through the irradiation window 250 and be irradiated outward.

Referring to FIG. 7, the distal end portion 232 may be disposed to be inclined with respect to the electron emission direction De. That is, a reflective surface of the distal end portion 232 may be disposed to be inclined with respect to the electron emission direction (e.g., the movement direction De of FIG. 2). In one embodiment, the distal end portion 232 may have a pyramid shape. The term "n-sided pyramid shape" refers to a three-dimensional shape having an n-sided polygonal base and triangular side surfaces, wherein apexes of the triangular side surfaces apart from the base meet at a single point positioned in a direction perpendicular to the base. In addition, in an n-sided pyramid, the term "number of pyramid sides" refers to n. When the distal end portion 232 has a pyramid shape, the number of pyramid sides may correspond to the number of the plurality of electron guns 220. The distal end portion 232 may include the same number of pyramid surfaces 232A as the number of pyramid sides. In one embodiment, angles of the respective pyramid surfaces 232A with respect to the longitudinal direction of the base end portion 231 may all be identical. When the angles formed by the respective pyramid surfaces 232A with respect to the longitudinal direction of the base end portion 231 are identical, X-rays emitted from the respective pyramid surfaces 232A may be irradiated onto a virtual circular path on a target surface. In another embodiment, the angles of the respective pyramid surfaces 232A with respect to the longitudinal direction of the base end portion 231 may all be different from one another. Since the angles formed by the respective pyramid surfaces 232A with respect to the longitudinal direction of the base end portion 231 are set differently from one another, X-rays emitted from the respective pyramid surfaces 232A may be irradiated onto positions desired by a user on the target surface.

In another embodiment, the distal end portion 232 may have a conical shape. When the distal end portion 232 has a conical shape, regardless of the number of the plurality of electron guns 220, electrons irradiated from the electron guns 220 may be reflected by the distal end portion 232 such that X-rays are irradiated onto an accurate position on a target surface.

In one embodiment, the case 240 and the irradiation window 250 may include all configurations and features substantially identical to those of the case (e.g., the case 140 of FIG. 1 and/or FIG. 2) and the irradiation window (e.g., the irradiation window 150 of FIG. 2) described with reference to the embodiment of FIGS. 1 and 2, and the same descriptions thereof may be equally applied to the present embodiment.

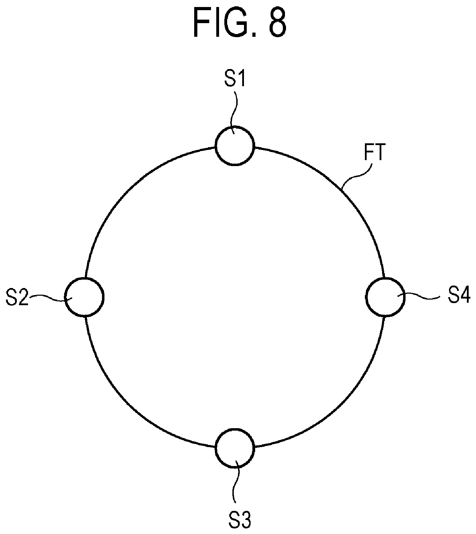

FIG. 8 is a schematic view illustrating a focal track FT of light source positions of the X-ray tube 200 according to an embodiment of the present disclosure. Specifically, FIG. 8 illustrates positions of light sources S1, S2, S3, and S4 on the focal track FT in a case including four electron guns (e.g., the electron guns 220 of FIGS. 5 to 7). For example, the first light source S1, the second light source S2, the third light source S3, and the fourth light source S4 may be disposed on a circular focal track FT. That is, the plurality of light sources S1, S2, S3, and S4 correspond to a set of points spaced apart from a center of the circular focal track FT by a predetermined distance. Since the plurality of light sources S1, S2, S3, and S4 are disposed on the circular focal track FT, X-ray images may be acquired from different positions, thereby obtaining images with minimized distortion when imaging a human body or an object having a three-dimensional shape.

Although the technical content of the present disclosure has been described above with reference to some embodiments and examples illustrated in the accompanying drawings, it should be understood that various substitutions, modifications, and alterations may be made by those skilled in the art without departing from the technical content and scope of the present disclosure. In addition, such substitutions, modifications, and alterations should be construed as falling within the scope of the appended claims.

## Claims

1. An X-ray tube comprising:
an electron gun;
an anode including a distal end portion onto which electrons emitted from the electron gun are irradiated and from which X-rays are emitted, the anode being surrounded by a conductive material;
a case including a first receptacle formed of the conductive material and connected to the electron gun, and a second receptacle coupled to the first receptacle and defining, together with the first receptacle, an internal space for accommodating the anode, the first receptacle including a side wall connected to the electron gun and a lower wall formed perpendicular to the side wall; and
an irradiation window formed in the lower wall of the first receptacle and configured to extract the X-rays emitted from the distal end portion to the outside of the case,
wherein a movement direction of the X-rays emitted from the distal end portion crosses a movement direction of the electrons emitted from the electron gun.

2. The X-ray tube of Claim 1, wherein, at a position corresponding to a receptacle coupling portion at which the first receptacle and the second receptacle are coupled to each other, a distance from an outer peripheral surface of the anode to the first receptacle is greater than a distance from the outer peripheral surface of the anode to the second receptacle.

3. The X-ray tube of Claim 2, wherein the anode includes:
a first portion located on a side of the second receptacle in the case;
a second portion located on a side of the lower wall of the first receptacle in the case; and
a third portion located between the first portion and the second portion,
wherein the first portion, the second portion, and the third portion are arranged side by side in a longitudinal direction, and a circumference of an outer peripheral surface of the third portion is smaller than a circumference of an outer peripheral surface of the first portion and a circumference of an outer peripheral surface of the second portion.

4. The X-ray tube of Claim 3, wherein the circumference of the outer peripheral surface of the first portion and the circumference of the outer peripheral surface of the second portion are identical.

5. The X-ray tube of Claim 1, wherein the second receptacle is formed of an insulator.

6. The X-ray tube of Claim 5, wherein the second receptacle is formed of ceramic.

7. The X-ray tube of Claim 1, wherein the irradiation window is integrally formed with the first receptacle.

8. The X-ray tube of Claim 1, wherein the irradiation window is formed of at least one selected from Be, Al, and Ti.

9. The X-ray tube of Claim 1, wherein the distal end portion is disposed to be inclined with respect to an emission direction of the electrons emitted from the electron gun.

10. An X-ray tube system comprising:
a plurality of X-ray tubes, each including:
an electron gun;
an anode including a distal end portion onto which electrons emitted from the electron gun are irradiated and from which X-rays are emitted, the anode being surrounded by a conductive material;
a case including a first receptacle formed of the conductive material and connected to the electron gun, and a second receptacle coupled to the first receptacle and defining, together with the first receptacle, an internal space for accommodating the anode, the first receptacle including a side wall connected to the electron gun and a lower wall formed perpendicular to the side wall; and
an irradiation window formed in the lower wall of the first receptacle and configured to extract the X-rays emitted from the distal end portion to the outside of the case;
a slit through which each of the X-rays passes and which adjusts an irradiation field of each of the X-rays; and
a detector configured to receive each of the X-rays emitted from the plurality of X-ray tubes,
wherein the plurality of X-ray tubes are arranged side by side on a plane parallel to the detector, and
wherein a movement direction of each of the X-rays crosses a movement direction of each of the electrons emitted from the electron gun.

11. The X-ray tube system of Claim 10, wherein the plurality of X-ray tubes includes three or more X-ray tubes configured to irradiate the detector with the respective X-rays in parallel with one another.

12. The X-ray tube system of Claim 11, wherein intensities of the respective X-rays are different from one another.

13. The X-ray tube system of Claim 12, wherein, among the three or more X-ray tubes, an intensity of X-rays emitted from an X-ray tube located between two other X-ray tubes is greatest.
